(19) Europäisches Patentamt
European Patent Office
Office européen des brevets

(11) Veröffentlichungsnummer: **0 116 929**
**B1**

(12) # EUROPÄISCHE PATENTSCHRIFT

(45) Veröffentlichungstag der Patentschrift: **02.10.85**

(51) Int. Cl.⁴: **C 07 C 143/11**, C 07 C 139/00, C 08 G 65/32, E 21 B 43/22

(21) Anmeldenummer: **84101455.8**

(22) Anmeldetag: **13.02.84**

(54) **Tributylphenol-ethersulfonate, deren Verwendung und Verfahren zur Herstellung von Ethersulfonaten.**

(30) Priorität: **17.02.83 DE 3305328**
**23.12.83 DE 3346676**

(43) Veröffentlichungstag der Anmeldung: **29.08.84 Patentblatt 84/35**

(45) Bekanntmachung des Hinweises auf die Patenterteilung: **02.10.85 Patentblatt 85/40**

(84) Benannte Vertragsstaaten: **CH DE FR GB IT LI NL SE**

(56) Entgegenhaltungen:
**EP - A - 0 026 932**
**DE - A - 2 748 721**
**US - A - 2 115 192**

(73) Patentinhaber: **HOECHST AKTIENGESELLSCHAFT, Postfach 80 03 20, D-6230 Frankfurt am Main 80 (DE)**

(72) Erfinder: **Wester, Norbert, Dr., Sachsenring 12, D-6238 Hofheim am Taunus (DE)**
Erfinder: **Uhl, Klaus, Dr., Am Dachsbau 1, D-6232 Bad Soden am Taunus (DE)**
Erfinder: **Gulden, Walter, Dr., Frankfurter Strasse 70, D-6238 Hofheim am Taunus (DE)**
Erfinder: **Schneider, Gerhart, Dr., Ziegelheck 3, D-6240 Königstein/Taunus (DE)**

**EP 0 116 929 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Alkyl- bzw. Alkylarylpolyethersulfonate sind wegen ihrer hohen Säure- und Alkalistabilität sowie ihrer ausgezeichneten Salzverträglichkeit von großem Interesse. Sie eignen sich gut für Reinigungszwecke in alkalischem und saurem Milieu. Aufgrund ihres hervorragenden Emulgiervermögens für Fette und Öle finden sie auch Anwendung z. B. in Kalkseifenformulierung und in der Emulsionspolymerisation. Darüber hinaus bieten sie sich als Hilfsmittel für die Erdölintensivförderung an.

Methoden zu ihrer Herstellung sind z. B. in DE 2 748 722, US 4 091 014 oder US 2 535 677 beschrieben. Bei einem anderen Herstellungsverfahren setzt man endständig halogenierte Alkyl- bzw. Alkylarylpolyether unter alkalischer Katalyse (EP 26 932) oder ohne Katalysator (US 2 148 432, US 2 115 192) bei höheren Temperaturen in wäßriger Lösung mit $Na_2SO_3$ zu den jeweiligen Ethersulfonaten um. Alkylsulfonate ohne Etherketten lassen sich z. B. ausgehend von Alkylhalogeniden durch entsprechende Umsetzung in Gegenwart einer quartären Ammoniumverbindung bei tieferen Temperaturen darstellen (DE 2 545 644).

Die Schwierigkeit beim Halogen-Sulfit-Austausch liegt darin, daß sich die beiden Reaktionspartner, halogenverschlossenes Oxethylat und $Na_2SO_3$, getrennt in zwei verschiedenen Phasen befinden. Die Phasentrennung wird noch durch die für die Reaktion erforderlichen hohen Reaktionstemperaturen, die zum Teil weit über den Trübungspunkten der eingesetzten Ether liegen, erheblich verstärkt. Dabei setzt als Nebenreaktion verstärkt die Hydrolyse der Halogenide zu den entsprechenden Alkoholen ein und als Folge davon sinkt die Ausbeute an gewünschtem Ethersulfonat, insbesondere bei höheren oxalkylierten Verbindungen, erheblich. Diesem Umstand versucht man beispielsweise durch heftiges Rühren (EP 26 932) oder durch den Einsatz von quartären Ammoniumverbindungen bei Alkylsulfonaten (DE 2 545 644) Rechnung zu tragen.

Es wurde nun überraschend gefunden, daß man bei dieser Reaktion durch Zugabe bestimmter Solubilisierungsmittel die Ausbeuten deutlich erhöhen, die Reaktionszeiten stark verkürzen und darüber hinaus besser hanbhabbare Produkte erhalten kann.

Gegenstand der Erfindung ist ein Verfahren zur Herstellung von Ethersulfonaten der Formel

$$R-(O-A)_n-SO_3M$$

worin R $C_8-C_{22}$-, vorzugsweise $C_{12}-C_{18}$-Alkyl oder Phenyl oder Naphthyl, die beide durch 1 bis 3 $C_1-C_{15}$-Alkyl, vorzugsweise $C_4-C_{12}$-Alkyl substituiert sein können, A vorzugsweise eine Ethylen- oder eine i-Propylengruppe, n eine Zahl von 1 bis 30, vorzugsweise 6 bis 20 und M Natrium oder Kalium bedeuten.

Das neue Verfahren besteht darin, daß man eine Verbindung der Formel

$$R-(O-A)_n-Hal$$

worin Hal ein Halogenatom bedeutet, in wäßriger Lösung mit Natrium- oder Kaliumsulfit umsetzt in Gegenwart von niederen Alkoholen, Polyolen, Mono- oder Poly-ethylenglykol oder deren Aklylethern.

Das Wesen der Erfindung beruht also auf dem Zusatz von speziellen Alkoholen als Solubilisierungsmittel. Für diesen Zweck kommen in Frage $C_1-C_4$-Alkanole, Polyole wie vorzugsweise Glycerin, Ethylenglykol oder Polyethylenglykole, insbesondere Di- bis Tetra-ethylenglykol und deren $C_1-C_4$-Alkyl, Mono- oder Di-ether. Insgesamt arbeitet man bevorzugt mit Mono-, Di- oder Triethylenglykol. Die Menge dieser Alkohole in dem Reaktionsansatz beträgt etwa 2 bis 60, vorzugsweise 10 bis 40 Gew.-% bezogen auf das Etherhalogenid. Die Reaktion wird durchgeführt bei Temperaturen von 120 bis 200° C, vorzugsweise 160 bis 180° C. Die Reaktionsdauer beträgt nur 2 bis 10, vorzugsweise 3 bis 6 Stunden. Das Sulfit wird als Natrium- oder Kaliumsulfit in einem leichten Überschuß eingesetzt. Als Reaktionsmedium dient Wasser, wobei die Konzentration der beiden Ausgangsverbindungen zusammen in dem Reaktionsansatz vorzugsweise ungefähr 40% beträgt. Zur Beschleunigung kann man auch übliche Substitutionskatalysatoren zugeben, wie zum Beispiel Alkalijodide.

Nach diesem Verfahren erhält man die Äthersulfonate in einer hohen Ausbeute von über 80%. Die anfallenden wäßrigen Lösungen der Äthersulfonate können ohne Isolierung der Äthersulfonate direkt weiter verwendet werden. Durch ihren Gehalt an den Alkoholen der oben beschriebenen Art zeigen diese Lösungen eine deutlich geringere Tendenz zur Bildung von Gelen.

Gegenstand der Erfindung sind weiterhin neue Tributylphenolethersulfonate der Formel

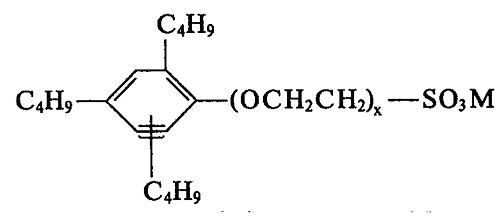

wobei x eine Zahl von 2 bis 20, vorzugsweise 2 bis 12, und M $Na^+$, $K^+$, $NH_4^+$ oder das Kation einer

Aminbase bedeuten. Diese Verbindungen können ebenfalls mit dem oben beschriebenen Verfahren hergestellt werden.

Die Verbindungen der obigen Formel leiten sich her vom Tributylphenol, vorzugsweise von technisch verfügbaren Mischungen aus 2,4,6-iso-Tributylphenol und einem geringeren Anteil von 2,4,5-iso-Tributylphenol, wobei diese Mischung weiterhin noch geringe Mengen iso-Dibutylphenol und iso-Tetrabutylphenol enthält. Ein Beispiel hierfür ist eine Mischung bestehend aus 1,4—2% 2,6-Iso-dibutylphenol, 0,3—0,8% 2,4-iso-dibutylphenol, 1,2—2,2% 2,5-Iso-dibutylphenol, 70—80% 2,4,6-Iso-Tributylphenol, 9—12% 2,4,5-Isotributylphenol und 6—7%, 2,4,5,6-Iso-tetrabutylphenol wobei der Gesamtanteil der drei Iso-dibutyl-Isomeren ca. 3% beträgt.

Die Ethersulfonate, besonders auch die Tributylphenolethersulfonate der obigen Formel sind Tenside, die sich durch Beständigkeit in einem weiten Temperatur- und ph-Bereich auszeichnen. Wäßrige Lösungen dieser Verbindungen erniedrigen die Oberflächenspannung an der Phasengrenze Tensidlösung/Luft auf Werte in der Größenordnung von 25 bis 30 $mNm^{-1}$ und die Grenzflächenspannung Öl/Tensidlösung auf Werte von $10^{-2}$ bis $10^{-4}$ $mNm^{-1}$ (nach Di Nouy). Aufgrund ihrer Wirksamkeit und Stabilität über einen weiten pH-Bereich eignen sich diese Verbindungen für alkalische und für saure Metallreinigungsverfahren. Sie sind außerdem wirksame Emulgiermittel für die Emulsions-Polymerisation und eignen sich als Stabilisatoren für Latex und andere Polymer-Emulsionen. Von besonderem Interesse ist auch die Verwendung dieser Verbindungen bei der Erdölförderung, wie etwa bei der Sondenstimulierung und Frac-behandlung von Erdöl-Lagerstätten. Hierbei wird die Umgebung einer Fördersonde durch eine Behandlung mit Säure oder durch Aufbrechen der Lagerstätte infolge der Anwendung sehr hohe Drucke für das Öl durchlässiger gemacht. Der Einsatz der Verbindung gemäß der vorliegenden Erfindung erhöht bei diesen Verfahren die Ölausbeute. In allen Fällen wird das Tensid im allgemeinen in Mengen von 0,01 bis 10, vorzugsweise 0,05 bis 3% eingesetzt.

## Beispiel 1

### Tributylphenol × 7,5 EO-Sulfonat

452 g (0,75 Mol) des durch Umsetzung von Tributylphenol plus 7,5 Mol Ethylenoxid (OH-Zahl: 96) mit $SOCl_2$ hergestellten Chlorids werden zusammen mit 104 g (0,825 Mol) $Na_2SO_3$, einer Spatelspitze NaJ, 83 g Diethylenglykol und 751 g Wasser in einen 2-l-Druckautoklaven gefüllt und bei einem Ausgangs-pH-Wert von 10—11 3 h bei 175° C gerührt. Man läßt auf ca. 50° C abkühlen und füllt ab. Das Reaktionsprodukt ist eine orangefarbene viskose klare Substanz mit einem Gehalt von 30,4% (entspricht 84% der theoretischen Menge an Sulfonat bezogen auf ein Molekulargewicht von 670).

## Beispiel 2

### Tributylphenol × 7,5 EO-Sulfonat

Entspricht Beispiel 1, jedoch wurde Triethylenglykol statt Diethylenglykol als Solubilisierungsmittel verwendet. Gehalt: 30,1% (83,2% d. Th.).

## Beispiel 3

### Tributylphenol × 10 EO-Sulfonat

385 g (0,5 Mol) der durch Umsetzung von Tributylphenol plus 10 Mol Ethylenoxid (OH-Zahl: 75) mit $SOCl_2$ hergestellten Chlorverbindung werden zusammen mit 69,3 g (0,55 Mol) $Na_2SO_3$, einer Spatelspitze NaJ, 69 g Diethylenglykol und 618 g Wasser in einen 2-l-Druckautoklaven gefüllt und bei einem Ausgangs-pH-Wert von 10—11 5 h bei 175° C Innentemperatur gerührt. Nach beendeter Reaktion läßt man auf ca. 50° C abkühlen und füllt ab. Das Reaktionsprodukt ist eine gelbe, viskose klare Flüssigkeit mit einem Gehalt von 29,0% (80,4% d. Th. bezogen auf ein Molekulargewicht von 824).

## Beispiel 4

### Tributylphenol × 10 EO-Sulfonat

Gleicher Ansatz wie in Beispiel 3, jedoch mit Triethylenglykol als Solubilisierungsmittel. Gehalt: 29,6% (82% d. Th.).

## Beispiel 5

### Tributylphenol × 13 EO-Sulfonat

440 g (0,5 Mol) des durch Umsetzung von Tributylphenol plus 13 Mol Ethylenoxid (OH-Zahl: 65) mit $SOCl_2$ hergestellten Chlorids werden zusammen mit 69,3 g (0,55 Mol) $Na_2SO_3$ einer Spatelspitze NaJ, 75 g Diethylenglykol und 690 g Wasser in einen 2-l-Rührautoklaven gefüllt und bei einem Anfangs-pH-Wert von 10—11 5 h bei einer Innentemperatur von 175°C gerührt. Man erhält eine braune, viskose Flüssigkeit mit einem Gehalt von 29,9% (80,2% d. Th. bezogen auf ein Molekulargewicht von 949).

## Beispiel 6

### Alfol 14 plus 3 Mol Ethylenoxid-Sulfonat
### (Alfol 14 = $C_{14}$-Alkohol)

350 g (0,94 Mol) des durch Umsetzung von Alfol 14 plus 3 EO (OH-Zahl: 159) mit Thionylchlorid hergestellten Chlorids werden zusammen mit 130,6 g (1,04 Mol) $Na_2SO_3$, einer Spatelspitze NaJ, 72 g Diethylenglykol und 650 g Wasser in ein 2-l-Druckautoklaven gefüllt und bei einem Anfangs-pH-Wert von 10—11 6 Stunden bei 175°C gerührt. Man läßt auf 60°C abkühlen und füllt ab. Das Endprodukt besitzt einen Gehalt von 31,4% (91,3% d. Th. bezogen auf ein Molekulargewicht von 439).

## Beispiele 7—9

Auf entsprechende Weise wurden hergestellt:

Talgfettalkohol plus 8 Mol Ethylenoxyd-Sulfonat (80,1% d. Th.)
Isotridecylalkohol plus 6 Mol Ethylenoxyd-Sulfonat (85,4% d. Th.)
Isotridecylalkohol plus 8 Mol Ethylenoxyd-Sulfonat (80,3% d. Th.)

## Vergleichsbeispiel 1
### (analog EP 26 932, Beispiel 6)

### Tributylphenol × 10 EO-Sulfonat

385 g (0,5 Mol) des durch Umsetzung mit $SOCl_2$ aus Tributylphenol plus 10 Mol Ethylenoxid (OH-Zahl: 75) hergestellten Chlorids, 69,3 g (0,55 Mol) $Na_2SO_3$, 3 g 50%ige Natronlauge und 680 g Wasser werden in einem 1-l-Druckautoklaven 20 h lang bei 160—165°C gerührt. Nach Reaktionsende läßt man auf 40°C abkühlen und füllt ab. Man erhält ein hochviskoses, gelbes Produkt mit einem Gehalt von 26,1% (72,1% d. Th. bezogen auf ein Molekulargewicht von 824).

## Vergleichsbeispiel 2
### (analog DT 2 545 644, Beispiel 1)

### Tributylphenol × 10 EO-Sulfonat

385 g (0,5 Mol) der durch Umsetzung von Thionchlorid mit Tributylphenol plus 10 Mol Ethylenoxid (OH-Zahl: 75) hergestellten Chlorverbindung, 74,3 g (0,59 Mol) $Na_2SO_3$, 4,3 g (0,013 Mol) Tetrabutylammoniumhydrogensulfat, 200 ml Ethanol und 500 ml Wasser werden 20 h lang unter Rückfluß (90°C) erhitzt. Der Sulfonat-Gehalt des nach Abdestillieren des Lösungsmittels erhaltenen Produktes war 2%, sein Gehalt an organisch gebundenem Chlor 4,7% (= Ausgangsverbindung).

## Vergleichsbeispiel 3
### (analog DT 2 545 644, Beispiel 1, mit oberer bevorzugter Menge an quaternärer Ammoniumverbindung)

77 g (0,1 Mol) des durch Umsetzung von Tributylphenol plus 10 Mol Ethylenoxid (OH-Zahl: 75) mit $SOCl_2$ hergestellten Chlorids werden zusammen mit 12,6 g (0,1 Mol) $Na_2SO_3$, 4,7 g (0,014 Mol) Tetrabutylammoniumhydrogensulfat, 200 ml Ethanol und 500 ml Wasser 20 Stunden unter Rückfluß (90°C) erhitzt. Das nach Verdampfen des Lösungsmittels erhaltene Produkt hatte einen Gehalt von 2%.

Die wesentlich schlechteren Ergebnisse der Vergleichsbeispiele unterstreichen die hervorragende Eignung des hier beanspruchten neuen Verfahrens zur Herstellung von Ethersulfonaten.

Zur Bestimmung der Wirksamkeit der Tributylphenolethersulfonate wird die in der US-Patentschrift 4 008 165 beschriebene Mikrokapillarentölungs-Methode und die Bestimmung der Grenzflächenspannung nach der Spinning-drop-interfacial-Tensiometer-Methode herangezogen.

Bei der Mikrokapillarentölung werden als Modell für den Porenraum der Lagerstätte Mikrokapillaren aus Glas der Firma Drummond Scientific Co./USA verwendet, die bei einem Volumen von 5 µl eine Länge von 30 mm und einen Durchmesser von 0,45 mm aufwiesen.

Die Mikrokapillaren werden an einem Ende abgeschmolzen, in einem Exsikator evakuiert und mit Rohöl gefüllt. Die Kapillaren werden in Tensidlösungen (Reagenzgläser), die im Wasserbad temperiert werden, mit der Öffnung nach oben senkrecht eingebracht und die Verdrängung des Öls wird in Abhängigkeit von der Zeit visuell registriert.

Mit Hilfe des folgenden Beurteilungsschemas kann die Wirksamkeit der Tenside in Abhängigkeit von dessen Konzentration, der Salzkonzentration, pH-Wert, Temperatur und Ölzusammensetzung bestimmt werden.

| Wert | |
|---|---|
| 9 | leer (30 mm) nach 10 Minuten |
| 8 | leer nach 1 Stunde |
| 7 | leer nach 3 Stunden |
| 6 | leer nach 20 Stunden |
| 5 | 16—25 mm Entleerung nach 20 Stunden |
| 4 | 9—15 mm Entleerung nach 20 Stunden |
| 3 | 4—8 mm Entleerung nach 20 Stunden |
| 2 | 1—3 mm Entleerung nach 20 Stunden |
| 1 | Spur Entleerung nach 20 Stunden |
| 0 | unverändert nach 20 Stunden |

Diese Methode bietet den Vorteil, das bei dem geringen Durchmesser der Mikrokapillaren, Viskosität und Dichte der Öle keinen großen Einfluß auf die Entölungswirkung ausüben und es möglich ist, mit Lagerstättenöl und Lagerstättenwasser zu arbeiten.

Nach Taber J. Petr. Techn. 3 (1969), S. 3—12 sind Tenside für die tertiäre Erdölgewinnung nur geeignet, wenn die Grenzflächenspannung an der Phasengrenze Öl/Salzwasser auf Werte kleiner $10^{-2}$ $mNm^{-1}$ erniedrigt wird. Für diese Bestimmung der Grenzflächenspannung an der Phasengrenze Öl/Wasser wird das vom Wade und Burkowsky entwickelte Spinning-Drop-Interface-Tensiometer verwendet. (M. Burkowsky und C. Marx: Über den Mechanismus des Tensidflutens in hochsalinaren Systemen; Erdöl-Erdgas-Zeitschrift 95 (1979), S. 17—25.

Die Methode beruht darauf, daß ein Öltropfen, der in eine um die horizontale Achse rotierende Kapillare gebracht wird, die eine Flüssigkeit (Salzwasser + Tensid) mit höherer Dichte enthält, deformiert wird. Der Tropfen wird gestreckt, bis ein Gleichgewicht der deformierten Kräfte und der Grenzflächenspannung erreicht wird.

Die Grenzflächenspannung errechnet sich nach Vonnegut (B. Vonnegut, Rev. Sci Instruments 13 (1942), S. 6—9) aus dem gemessenen Öltropfendurchmesser R, der Rotationsgeschwindigkeit W und dem Dichteunterschied $\Delta$d nach folgender Formel:

$$\gamma^1/_2 = \frac{\Delta d \cdot W^2 \cdot R^3}{4}.$$

Die mit diesen Methoden gemessenen Werten sind in den folgenden Tabellen zusammengefaßt. Es wurden in allen Fällen jeweils 1%ige wäßrige Lösungen der Tenside verwendet. Als Vergleichsprodukte gemäß dem Stand der Technik wurden die Ethersulfonate von Nonylphenol und Di-Nonylphenol mit getestet.

I. Test mit Lagerstättenöl K

Salzgehalt: 180 g/l NaCl, 20 g/l $CaCl_2$, pH 8,5
Temperatur: 40°C

| | Grenzflächen-spannung $[mNm^{-1}]$ | Mikrokapillar-entölung |
|---|---|---|
| Tributyl 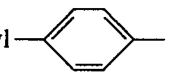 $O(CH_2CH_2O)_8CH_2CH_2SO_3Na$ | $2 \cdot 10^{-3}$ | 6 |
| Nonyl 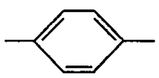 $O(CH_2CH_2O)_8CH_2CH_2SO_3Na$ | $3 \cdot 10^{-2}$ | 0 |
| Di-Nonyl 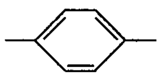 $O(CH_2CH_2O)_6CH_2CH_2SO_3Na$ | $8 \cdot 10^{-2}$ | 0 |

II. Test mit Lagerstättenöl K

Salzgehalt: 100 g/l NaCl, pH 8,5
Temperatur: 40°C

| | Grenzflächenspannung $[mNm^{-1}]$ |
|---|---|
| Tributyl 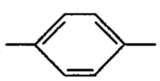 $O(CH_2CH_2O)_4CH_2CH_2SO_3Na$ | $1 \cdot 10^{-2}$ |
| Di-Nonyl 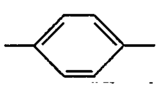 $O(CH_2CH_2O)_6CH_2CH_2SO_3Na$ | $8 \cdot 10^{-2}$ |

III. Test mit einem paraffinischen Öl

Salzgehalt: 135 g/l NaCl, 15 g/l $CaCl_2$, pH 8,5
Temperatur: 60°C

| | Grenzflächen-spannung $[mNm^{-1}]$ | Mikrokapillar-entölung |
|---|---|---|
| Tributyl 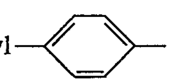 $O(CH_2CH_2O)_6CH_2CH_2SO_3Na$ | $1 \cdot 10^{-3}$ | 8 |
| Nonyl 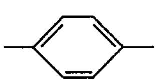 $O(CH_2CH_2O)_6CH_2CH_2SO_3Na$ | $3 \cdot 10^{-2}$ | 0 |
| Nonyl 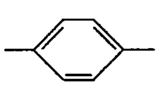 $O(CH_2CH_2O)_6CH_2CH_2CH_2SO_3Na$ | $8 \cdot 10^{-2}$ | 0 |
| Di-Nonyl 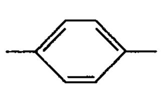 $O(CH_2CH_2)_6CH_2CH_2SO_3Na$ | $4 \cdot 10^{-2}$ | 0 |

IV. Test mit einem aromatischen Öl

Salzgehalt: 70 g/l NaCl, 30 g/l $CaCl_2$, pH 6,5
Temperatur: 80°C

| | Grenzflächenspannung [$mNm^{-1}$] |
|---|---|
| Tributyl 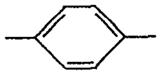 $O(CH_2CH_2O)_8CH_2CH_2SO_3Na$ | $1{,}3 \cdot 10^{-2}$ |
| Nonyl 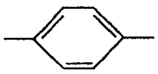 $O(CH_2CH_2O)_8CH_2CH_2SO_3Na$ | $3{,}8 \cdot 10^{-2}$ |
| Di-Nonyl 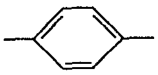 $O(CH_2CH_2O)_8CH_2CH_2SO_3Na$ | $8 \cdot 10^{-2}$ |

V. Test mit einem aromatischen Öl

Salzgehalt: 135 g/l NaCl, 15 g/l $CaCl_2$, pH 8,5
Temperatur: 40°C

| | Grenzflächen-spannung [$mNm^{-1}$] | Mikrokapillar-entölung |
|---|---|---|
| Tributyl 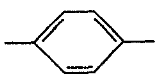 $O(CH_2CH_2O)_6CH_2CH_2SO_3Na$ | $3 \cdot 10^{-3}$ | 8 |
| Nonyl 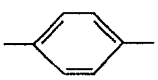 $O(CH_2CH_2O)_6CH_2CH_2SO_3Na$ | $1 \cdot 10^{-2}$ | 0 |
| Nonyl 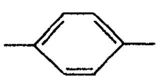 $O(CH_2CH_2O)_6CH_2CH_2CH_2SO_3K$ | $1 \cdot 10^{-1}$ | 0 |
| Di-Nonyl 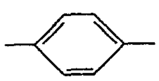 $O(CH_2CH_2O)_6CH_2CH_2SO_3Na$ | $8 \cdot 10^{-2}$ | 0 |

VI. Test mit einem aromatischen Öl

Salzgehalt: 105 g/l NaCl, 45 g/l $CaCl_2$, pH 8,5
Temperatur: 40°C

| | Grenzflächen-spannung $[mNm^{-1}]$ | Mikrokapillar-entölung |
|---|---|---|
| Tributyl— 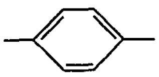 —$O(CH_2CH_2O)_6CH_2CH_2SO_3Na$ | $2 \cdot 10^{-3}$ | 8 |
| Nonyl— 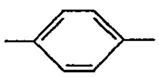 —$O(CH_2CH_2O)_6CH_2CH_2SO_3Na$ | $1 \cdot 10^{-2}$ | 0 |
| Nonyl— 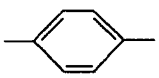 —$O(CH_2CH_2O)_6CH_2CH_2CH_2SO_3K$ | $8 \cdot 10^{-2}$ | 0 |
| Di-Nonyl— 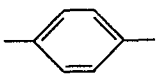 —$O(CH_2CH_2O)_6CH_2CH_2SO_3Na$ | $7 \cdot 10^{-2}$ | 0 |

VII. Test mit einem naphthenischen Öl

Salzgehalt: 135 g/l NaCl, 15 g/l $CaCl_2$, pH 8,5
Temperatur: 60°C

| | Grenzflächen-spannung $[mNm^{-1}]$ | Mikrokapillar-entölung |
|---|---|---|
| Tributyl— 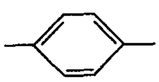 —$O(CH_2CH_2O)_6CH_2CH_2SO_3Na$ | $9 \cdot 10^{-4}$ | 8 |
| Nonyl— 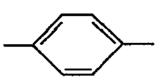 —$O(CH_2CH_2O)_6CH_2CH_2SO_3Na$ | $3 \cdot 10^{-2}$ | 0 |
| Nonyl— 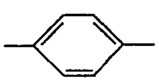 —$O(CH_2CH_2O)_6CH_2CH_2CH_2SO_3Na$ | $1 \cdot 10^{-1}$ | 0 |
| Di-Nonyl— 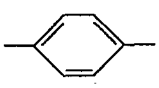 —$O(CH_2CH_2O)_6CH_2CH_2SO_3Na$ | $9 \cdot 10^{-2}$ | 0 |

VIII. Test mit einem naphthenischen Öl

Salzgehalt: 140 g/l NaCl, 60 g/l $CaCl_2$, pH 6,5
Temperatur: 60°C

| | Grenzflächen-spannung $[mNm^{-1}]$ | Mikrokapillar-entölung |
|---|---|---|
| 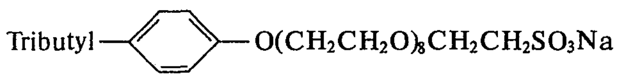 | $2 \cdot 10^{-3}$ | 8 |
| 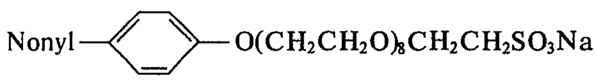 | $3 \cdot 10^{-2}$ | 0 |
| 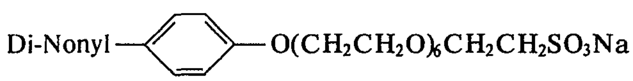 | $6 \cdot 10^{-2}$ | 0 |

IX. Test mit einem paraffinischen (1) naphthenischen (2) und aromatischen Öl (3)

Salzgehalt: jeweils 150 g/l NaCl, pH 6,5
Temperatur: 80°C

| | Grenzflächenspannung $[mNm^{-1}]$ | | |
|---|---|---|---|
| | (1) | (2) | (3) |
| 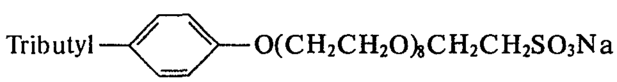 | $1{,}3 \cdot 10^{-2}$ | $4{,}2 \cdot 10^{-3}$ | $1{,}9 \cdot 10^{-3}$ |
| 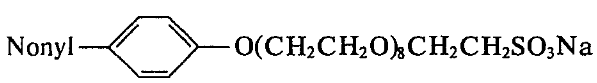 | $9{,}4 \cdot 10^{-2}$ | $3{,}5 \cdot 10^{-2}$ | $3{,}8 \cdot 10^{-2}$ |
| 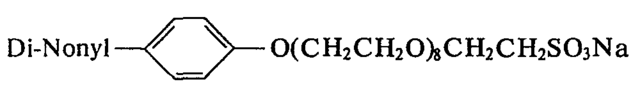 | – | – | $8 \cdot 10^{-2}$ |

Die Tabellen zeigen, daß die iso-Tributylphenolethersulfonate bei unterschiedlichen Salzgehalten (50—200 g/l) den bereits bekannten Verbindungen in der Ölmobilisierung überlegen sind.

Die Ethersulfonate können auch in Kombinationen mit anderen anionischen Tensiden, wie z. B. Petrolsulfonaten, sec. Alkansulfonaten, $\alpha$-Olefinsulfonaten, Dodecylbenzolsulfonaten und nichtionischen Tensiden wie Alkyl- bzw. Alkylphenolpolyglykolethern eingesetzt werden. Als weitere Zusätze kommen Alkohole und Glykolether in Frage. Die Viskosität des Flutwassers kann außerdem durch Polymere, wie z. B. Hydroxiethylcellulose, Polyacrylamide oder Polysaccaride erhöht werden.

**Patentansprüche**

1. Verfahren zur Herstellung von Ethersulfonaten der Formel

$$R-(O-A)_n-SO_3M$$

worin R $C_8-C_{22}$-Alkyl oder Phenyl oder Naphthyl, die beide durch 1 bis 3 $C_1-C_{15}$-Alkyl substituiert sein können, A eine Ethylen- oder eine i-Propylengruppe, n eine Zahl von 1 bis 30 und M Natrium oder Kalium bedeuten, dadurch gekennzeichnet, daß man eine Verbindung der Formel

$$R-(O-A)_n-Hal$$

worin Hal ein Halogenatom bedeutet, in wäßriger Lösung mit Natrium- oder Kaliumsulfit umsetzt in Gegenwart von niederen Alkoholen, Polyolen, Mono- oder Polyethylenglykol oder deren Alkylethern.

2. Verfahren nach Anspruch 1, dadurch gekennzeichnet, daß man Ethersulfonate der Formel

$$R-(O-A)_n-SO_3M$$

herstellt worin R $C_{12}-C_{18}$-Alkyl oder Phenyl oder Naphthyl, die beide durch 1 bis 3 $C_4-C_{12}$-Alkyl substituiert sein können, A eine Ethylengruppe, n eine Zahl von 6 bis 20 M Natrium oder Kalium bedeutet.

3. Verfahren nach Anspruch 1, dadurch gekennzeichnet, daß man die Reaktion in Gegenwart von Mono-, Di-, Tri- und Tetraethylenglykol oder deren $C_1-C_4$-Alkyl Mono- oder Diether oder Glycerin durchführt.

4. Verfahren nach Anspruch 1, dadurch gekennzeichnet, daß man die Reaktion in Gegenwart von Substitutionskatalysatoren wie NaJ oder KJ bei einem Anfangs-pH-Wert von 10—11 durchführt.

5. Tributylphenol-ethersulfonate der Formel

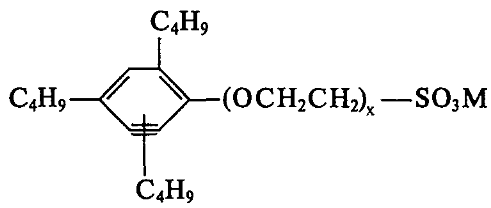

wobei x eine Zahl von 2 bis 20 und M $Na^+$, $K^+$, $NH_4^+$ oder das Kation einer Aminbase bedeuten.

6. Verwendung der Tributylphenyl-ethersulfonate nach Anspruch 5 bei der tertiären Erdölförderung.

**Claims**

1. A process for preparing sulfonated alkoxylates of the formula

$$R-(O-A)_n-SO_3M$$

in which R denotes $C_8-C_{22}$-alkyl or phenyl or naphthyl, both of which can be substituted by 1 to 3 $C_1-C_{15}$-alkyl, A denotes an ethylene or an isopropylene group, n denotes a number from 1 to 30 and M denotes sodium or potassium, which comprises reacting a compound of the formula

$$R-(O-A)_n-Hal$$

in which Hal denotes a halogen atom, in aqueous solution with sodium sulfite or potassium sulfite in the presence of lower alcohols, polyols, monoethylene glycol, polyethylene glycol or alkyl ethers thereof.

2. The process as claimed in claim 1, wherein the sulfonated alkoxylates prepared have the formula

$$R-(O-A)_n-SO_3M$$

in which R is $C_{12}-C_{18}$-alkyl or phenyl or naphthyl, both of which can be substituted by 1 to 3 $C_4-C_{12}$-alkyl, A denotes an ethylene group, n denotes a number from 6 to 20, and M denotes sodium or potassium.

3. The process as claimed in claim 1, wherein the reaction is carried out in the presence of monoethylene, diethylene, triethylene and tetraethylene glycol or their $C_1-C_4$-alkyl monoethers or diethers, or glycerol.

4. The process as claimed in claim 1, wherein the reaction is carried out at a starting pH of 10—11 in the presence of substitution catalysts, such as NaI or KI.

5. A sulfonated tributylphenol ethoxylate of the formula

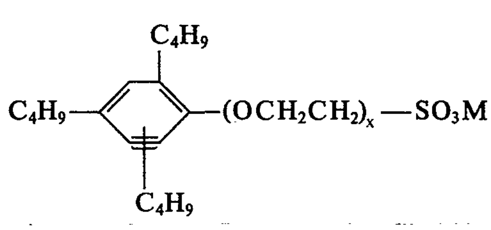

where x denotes a number from 2 to 20, and M denotes $Na^+$, $K^+$, $NH_4^+$ or the cation of an amine base.

6. Use of a sulfonated tributylphenyl ethoxylate as claimed in claim 5, in the tertiary production of crude oil.

**Revendications**

1. Procédé de préparation d'éther-sulfonates répondant à la formule:

$$R-(O-A)_n-SO_3M$$

dans laquelle R représente un alkyle en $C_8-C_{22}$, un phényle ou un naphtyle, chacun de ces deux derniers radicaux pouvant porter de 1 à 3 alkyles en $C_1-C_{15}$, A représente un radical éthylène ou isopropylène, n un nombre de 1 à 30 et M le sodium ou le potassium, procédé caractérisé en ce qu'on fait réagir un composé répondant à la formule:

$$R-(O-A)_n-Hal$$

dans laquelle Hal représente un atome d'halogène, en solution aqueuse, avec le sulfite de sodium ou de potassium, en présence d'alcools inférieurs, de polyols, de mono- ou de poly-éthylène-glycols ou de leurs éthers alkyliques.

2. Procédé selon la revendication 1, caractérisé en ce qu'on prépare des éther-sulfonates répondant à la formule:

$$R-(O-A)_n-SO_3M$$

dans laquelle R représente un alkyle en $C_{12}-C_{18}$, un phényle ou un naphtyle, chacun de ces deux derniers radicaux pouvant porter de 1 à 3 alkyles en $C_4-C_{12}$, A représente un radical éthylène, n un nombre de 6 à 20 et M le sodium ou le potassium.

3. Procédé selon la revendication 1, caractérisé en ce qu'on effictue la réaction en présence de mono-éthylène-glycol, de di-éthylène-glycol, de tri-éthylène-glycol et de tétra-éthylène-glycol, d'un de leurs mono- ou diéthers d'alkyles en $C_1-C_4$, ou de glycérol.

4. Procédé selon la revendication 1, caractérisé en ce qu'on effectue la réaction en présence de catalyseurs de substitution, tels que NaI ou KI,à un pH de départ de 10 à 11.

5. Tributyl-phénol-éther-sulfonates répondant à la formule:

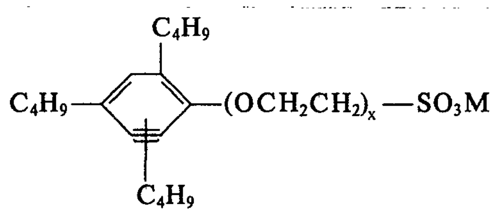

dans laquelle x désigne un nombre de 2 à 20 et M représente $Na^+$, $K^+$, $NH_4^+$ ou le cation d'une amine basique.

6. Application des tributylphénol-éther-sulfonates selon la revendication 5 dans l'exploitation tertiaire du pétrole.